# EUROPÄISCHE PATENTANMELDUNG

(11) **EP 0 629 854 A2**
(43) Veröffentlichungstag der Anmeldung: **21.12.1994**
(21) Anmeldenummer: 94250145.3
(22) Anmeldetag: 08.06.1994
(51) Int. Cl.: G01N 27/49, G01N 33/00

(54) **Elektrochemischer Gasdetektor**

(30) Priorität: 14.06.1993 DE 4319573
(71) Anmelder: MANNESMANN Aktiengesellschaft, D-40213 Düsseldorf (DE)
(72) Erfinder: Braden,Christoph,Dr. Dipl.-Phys., D-50937 Köln (DE)
(74) Vertreter: Meissner, Peter E., Dipl.-Ing.

(57) **Zusammenfassung**

Der Gasdetektor besteht im wesentlichen aus einem elektrochemischen Sensor mit einer Eintrittsblende (2), durch die das Meßgas an die Sensoroberfläche (9) diffundiert und dort ein mit der Gaskonzentration korreliertes elektrisches Meßsignal erzeugt. Die Eintrittsblende (2) wird aus einer auf einem Trägergerüst (11) aufgebrachten Keramikmembran mit einer Dicke von 0,1 µm bis 20 µm und mindestens einer Durchtrittsöffnung (12) von 0,1 µm bis 200 µm Durchmesser gebildet. Diese Keramikmembran (10) ist gegenüber einer wesentlich größeren Öffnung (13) im Trägergerüst (11) angeordnet. Die Dicke der Keramikmembran beträgt 0,1 µm bis 10 µm.

## Beschreibung

Die Erfindung betrifft einen Gasdetektor, der aus einem Meßkopf mit einem elektrochemischen Sensor und einer Eintrittsblende besteht.

Elektrochemische Gassensoren werden zur Messung von Gasspuren hauptsächlich von toxischen Gasen eingesetzt. Der Meßeffekt beruht darauf daß die durch die Eintrittsblende eintretenden und auf die Sensoroberfläche auftreffenden Meßgasmoleküle durch die Diffusionsschicht einer Gasdiffusionselektrode diffundieren und aufgrund einer elektrochemischen Reaktion an der Katalysatorschicht der Gasdiffusionselektrode ionisiert und abtransportiert werden. Die gebildeten Ionen wandern durch den Elektrolyt zur Gegenelektrode und erzeugen in einem äußeren Schließungskreis ein Stromsignal, das nach Verstärkung angezeigt wird und gegebenenfalls (bei Überschreibung einer vorgegebenen Grenzkonzentration) für eine Alarmfunktion ausgenutzt wird. Zwischen der Gaskonzentration und dem elektrochemischen Strom besteht nur dann ein linearer Zusammenhang, wenn alle eintreffenden Moleküle abtransportiert werden, d.h. wenn die Gaskonzentration an der umsetzenden Elektrode Null ist. In diesem Fall wird der sogenannte Diffusionsgrenzstrom erreicht. Bei hohen Gaskonzentrationen oder bei sehr kleinen Sensoroberflächen (z.B. bei einer Miniatur-Gasmeßzelle) kann der Fall eintreten, daß die elektrochemische Aktivität der Sensorelektrode (Arbeitselektrode) nicht mehr ausreicht, um die gesamte an der Sensoroberfläche eintreffende Gasmenge umzusetzen. In solchen Fällen muß der einfallende Meßgasstrom durch eine Lochblende (Eintrittsblende) reduziert bzw. gedrosselt werden, um eine vollständige elektrochemische Gasumsetzung zu gewährleisten und sicherzustellen daß der Sensor im Bereich des Diffusionsgrenzstromes arbeitet. Mit der Eintrittsblende kann auch der Meßbereich des Gasdetektors festgelegt werden. Gemäß EP-A-16423 sind z.B. auswechselbare Blendenköpfe mit abgestuften Öffnungsquerschnitten vorgesehen, um verschiedene Meßbereiche einzustellen. Eine weitere Möglichkeit den Gasstrom zu reduzieren besteht darin, daß die Sensoroberfläche durch eine geschlossenporige Diffusionsfolie abgedeckt wird. Damit kann zwar eine hinreichend große Reduzierung des Gasstromes erreicht werden; man handelt sich aber dadurch den Nachteil einer starken Temperaturabhängigkeit ein, da die Diffusionskonstante für die Gasdiffusion durch die Folie hindurch stark temperaturabhängig ist. Ferner kann die Gasstromreduzierung auch mit Hilfe eines porösen Keramikkörpers erfolgen. Ein vorgeschalteter Keramikkörper mit Mikroporen führt zwar zu einer ausreichend großen Gasstromreduzierung bei hinreichend kleinem Temperaturkoeffizienten, hat aber den Nachteil, daß die Porosität des Keramikkörpers in der Regel festliegt, so daß andere Meßbereiche schwer zu realisieren sind und die Reproduzierbarkeit in Frage gestellt ist (siehe z.B. US 48 10 352).

Der Erfindung liegt die Aufgabe zugrunde, einen elektrochemischen Gassensor in Verbindung mit einer Eintrittsblende zu schaffen, die sich fertigungstechnisch günstig in großen Stückzahlen mit hoher Reproduzierbarkeit herstellen läßt und einen niedrigen Temperaturkoeffizient aufweist.

Diese Aufgabe wird erfindungsgemäß dadurch gelöst, daß die Eintrittsblende aus einer auf einer Trägerschicht aufgebrachten Keramikmembran mit einer Dicke von 0,1 µm bis 20 µm und mindestens einer Öffnung von 0,1 µm bis 200 µm besteht, die gegenüber einer wesentlich größeren Öffnung in der Trägerschicht angeordnet ist. Vorzugsweise beträgt die Dicke der Keramikmembran 0,1 µm bis 10 µm.

Vorteilhaft bildet die Keramikmembran mit der Trägerschicht eine festhaftende Verbundstruktur. Eine solche Verbundstruktur erhält man z.B. dadurch, daß man die Keramikmembran durch chemische Umwandlung oder Reaktion auf der Oberfläche der Trägerschicht aufwachsen läßt.

Gemäß einer bevorzugten Ausführungsform besteht die Trägerschicht aus einem Silizium-Wafer. Die Keramikmembran kann dann vorteilhaft aus Siliziumnitrit, Aluminiumoxid, Siliziumoxid, Wolframoxid oder Titandioxid bestehen.

Der erfindungsgemäße Gasdetektor mit den neuen Mikrokeramikblenden ist in besonderem Maße zur Messung von Sauerstoff in der Umgebungsluft geeignet, da der hier übliche Meßbereich von typisch 16 bis 26 % eine sehr starke Reduzierung des Gasstromes zum Sensor erfordert.

Mit der Erfindung werden folgende Vorteile erzielt
- zur Herstellung der neuartigen Keramikblenden können die aus der Halbleitertechnik bekannten Mikrostrukturierungstechnologien benutzt werden. Auf diese Weise können die sehr dünnen Keramikmembranen mit ausgesprochen feinen Blendenöffnungen im Verbund mit einem Trägergerüst mit hoher Reproduzierbarkeit und kostengünstig in großen Stückzahlen hergestellt werden. Durch die Verbundstruktur wird auch die mechanische Stabilität der feinen Keramikmembran sichergestellt.
- Die neuen Mikrokeramikblenden haben einen sehr geringen Temperaturkoeffizient, so daß der Reduzierungsfaktor für das Meßgas und damit der Meßbereich über einen großen Temperaturbereich konstant ist. Darüber hinaus besitzen die Mikrokeramikblenden eine sehr gute chemische Resistenz gegenüber aggressiven Umwelteinflüssen, so daß eine hohe Langzeitstabilität erreicht wird.

Im folgenden wird die Erfindung anhand eines in der Zeichnung dargestellten Ausführungsbeispieles naher beschrieben. Es zeigen
Fig. 1 schematisch den Aufbau des elektrochemischen Gasdetektors und
Fig. 2 einen vergrößerten Ausschnitt im Bereich der Eintrittsblende.

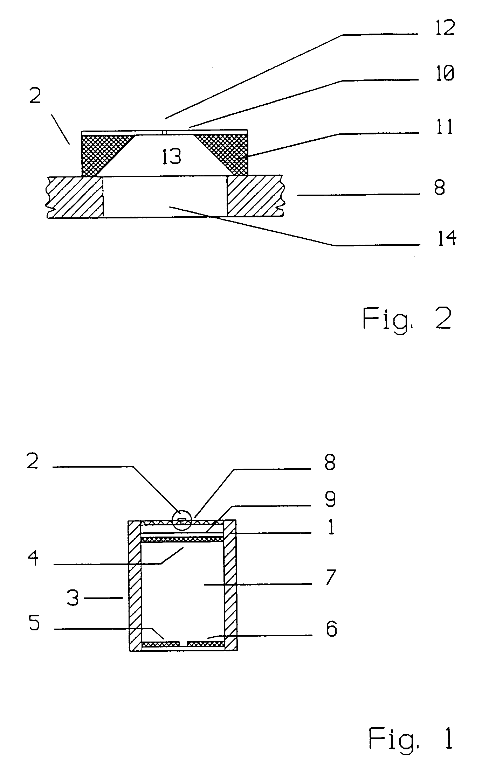

Der Gasdetektor gemäß Fig. 1 besteht aus einem Gehäuse 1 mit einer Eintrittsblende 2 und einem elektrochemischen Dreielektrodensensor 3. Der Dreielektrodensensor weist auf der einen Seite eine Arbeitselektrode 4 und auf der gegenüberliegenden Seite eine Gegenelektrode 5 und Bezugselektrode 6 auf. Zwischen der Arbeitselektrode 4 und den Elektroden 5 und 6 befindet sich der Meßzellenelektrolyt 7. Das zu untersuchende Meßgas diffundiert durch die in einer Adapterplatte 8 untergebrachte Eintrittsblende 2 hindurch und erreicht dann die Sensoroberfläche 9.

Fig. 2 zeigt die Eintrittblende 2 im Detail. Die auf der Adapterplatte 8 angeordnete Einzelblende besteht aus einer 10 µm dicken, perforierten Keramikmembran aus Aluminiumoxid, die auf ein Siliziumträgergerüst 11 aufgebracht ist. Die Keramikmembran 10 überdeckt eine wesentlich größere Öffnung 13 im Siliziumträgergerüst 11. Durchmesser und Form der Öffnung 13 sind unkritisch solange nur die Bedingung erfüllt ist, daß der Durchmesser der Öffnung 13 sehr viel größer ist als der Durchmesser der Blendenöffnung 12. Die Öffnung 13 steht mit einem durch die Adapterplatte 8 hindurchgehenden Eintrittskanal 14 in Verbindung, dessen Durchmesser ebenfalls sehr viel größer ist als der. Durchmesser der Blendenöffnung 12. Das Meßgas diffundiert also nacheinander durch die Blendenöffnung 12, die Öffnung 13 und den anschließenden Eintrittskanal 14, wobei der die Empfindlichkeit und den Meßbereich bestimmende Eintrittsquerschnitt (Reduzierung des Gasstromes) einzig und allein durch die Blendenöffnung 12 festgelegt wird.

Bei der Herstellung der Eintrittsblende 2 wird in einem ersten Schritt die Keramikmembran 12 durch Oxidation einer auf einem Silizium-Wafer aufgedampften Aluminiumschicht hergestellt. Auf diese Weise bildet die Keramikmembran mit ihrer Trägerschicht bzw. mit dem Siliziumträgergerüst 11 eine festhaftende Verbundstruktur. Dann werden in zwei Schritten mit Hilfe einer aus der Halbleitertechnologie bekannten photolitographischen Ätztechnik die Öffnung 12 in der Keramikmembran 10 und die Öffnung 13 im Silizium-Wafer eingeätzt. Anstelle einer Blendenöffnung 12 können selbstverständlich auch mehrere Eintrittsöffnungen vorgesehen werden. Die Abmessungen des Siliziumträgergerüsts 11 liegen typischerweise in einem Bereich von 0,5 x 0,5 mm² bis 3 x 3 mm², so daß aus einem Standard Silizium-Wafer bis über 15000 Blendenöffnungen hergestellt werden können.

Wie in Fig. 2 gezeigt, wird eine Blendeneinheit 10, 11, 12, 13 jeweils auf eine Adapterplatte 8 aufgeklebt. Die Adapterplatte 8 mit der Bohrung 14 (Eintrittskanal) kann aus Kunststoff bestehen. Das so montierte Bauteil wird anschließend in das Gehäuse 1 des Gasdetektors eingesetzt (siehe Fig. 1).

## Patentansprüche

1. Gasdetektor bestehend aus einem Meßkopf mit einem elektrochemischen Sensor und einer Eintrittsblende (2), durch die das Meßgas an die Sensor-Oberfläche (9) diffundiert, dadurch gekennzeichnet, daß die Eintrittsblende (2) aus einer auf einem Trägergerüst (11) aufgebrachten Keramikmembran (10) mit einer Dicke von 0,1 µm bis 20 µm und mindestens einer Öffnung (12) von 0,1 µm bis 200 µm besteht, die gegenüber einer wesentlich größeren Öffnung (13) im Trägergerüst (11) angeordnet ist.

2. Gasdetektor nach Anspruch 1, dadurch gekennzeichnet, daß die Dicke der Keramikmembran (10) 0,1 µm bis 10 µm beträgt.

3. Gasdetektor nach Anspruch 1 bis 2, dadurch gekennzeichnet, daß die Keramikmembran (10) mit dem Trägergerüst (11) eine festhaftende Verbundstruktur bildet.

4. Gasdetektor nach Anspruch 1 bis 3, dadurch gekennzeichnet, daß das Trägergerüst (11) aus einem Silizium-Wafer hergestellt ist.

5. Gasdetektor nach Anspruch 1 bis 4, dadurch gekennzeichnet, daß die Keramikmembran (1o) aus Si₃N₄, Al₂O₃, SiO₂, WO₂ oder TiO₂ besteht.

6. Verwendung des Gasdetektors nach Anspruch 1 bis 5 zur Messung von Sauerstoff.
